# EUROPEAN PATENT SPECIFICATION

(11) **EP 4 324 326 B1**
(45) Date of publication and mention of the grant of the patent: **06.11.2024**
(21) Application number: 22460037.9
(22) Date of filing: 17.08.2022
(51) Int. Cl.: A01K 67/027, A61K 31/506, A61K 31/4439, A61K 31/5025, A61P 9/00

(54) **MOUSE MODEL FOR HEART FAILURE WITH PRESERVED EJECTION FRACTION AND METHOD OF OBTAINING THIS MOUSE MODEL**
MAUSMODELL FÜR HERZINSUFFIZIENZ MIT KONSERVIERTER AUSSTOSSFRAKTION UND VERFAHREN ZUM ERHALT DIESES MAUSMODELLS
MODÈLE MURIN POUR INSUFFISANCE CARDIAQUE AVEC FRACTION D'ÉJECTION PRÉSERVÉE ET PROCÉDÉ D'OBTENTION DE CE MODÈLE MURIN

(43) Date of publication of application: 21.02.2024
(73) Proprietor: Uniwersytet Jagiellonski, 31-007 Krakow (PL)
(72) Inventor: Kwiatkowski, Grzegorz, 30-523 Krakow (PL); Chlopicki, Stefan, 31-138 Krakow (PL); Marczyk, Brygida, 32-095 Iwanowice (PL); Bar, Anna, 30-724 Krakow (PL)
(74) Representative: Czarnik, Maciej

(56) References cited:
- KERKELÄ RISTO ET AL: "Cardiotoxicity of the cancer therapeutic agent imatinib mesylate", NATURE MEDICINE, NATURE PUBLISHING GROUP US, NEW YORK, vol. 12, no. 8, 23 July 2006 (2006-07-23), pages 908 - 916, XP036981905, ISSN: 1078-8956, [retrieved on 20060723], DOI: 10.1038/NM1446
- JACKSON SAMUEL J ET AL: "Does age matter? The impact of rodent age on study outcomes", LABORATORY ANIMALS., vol. 51, no. 2, April 2017 (2017-04-01), GB, pages 160 - 169, XP093018638, ISSN: 0023-6772, Retrieved from the Internet <URL:https://www.ncbi.nlm.nih.gov/pmc/articles/PMC5367550/pdf/10.1177_0023677216653984.pdf> DOI: 10.1177/0023677216653984
- JACOBS JOSHUA A. ET AL: "Differentiating pulmonary hypertension associated with protein kinase inhibitors", PULMONARY CIRCULATION 2012 APR-JUN, vol. 12, no. 2, April 2022 (2022-04-01), XP093018711, ISSN: 2045-8940, Retrieved from the Internet <URL:https://onlinelibrary.wiley.com/doi/full-xml/10.1002/pul2.12075> DOI: 10.1002/pul2.12075
- "Proceedings of the RAMI Section of Biomedical Sciences Annual Meetings, 2013- 2015", IRISH JOURNAL OF MEDICAL SCIENCE, CAHILL, DUBLIN, IE, vol. 185, no. 1, 21 January 2016 (2016-01-21), pages 1 - 55, XP035776079, ISSN: 0021-1265, [retrieved on 20160121], DOI: 10.1007/S11845-015-1351-0
- CHOU CHUN ET AL: "Modeling heart failure with preserved ejection fraction in rodents: Where do we stand?", FRONTIERS IN DRUG DISCOVERY, vol. 2, August 2022 (2022-08-01), XP093018717, DOI: 10.3389/fddsv.2022.948407
- RASCHI E ET AL: "Anticancer drugs and cardiotoxicity: Insights and perspectives in the era of targeted therapy", PHARMACOLOGY & THERAPEUTICS, ELSEVIER, GB, vol. 125, no. 2, February 2010 (2010-02-01), pages 196 - 218, XP026894625, ISSN: 0163-7258, [retrieved on 20091027], DOI: 10.1016/J.PHARMTHERA.2009.10.002
- SINGH ANAND PRAKASH ET AL: "Cardiotoxicity of the BCR-ABL1 tyrosine kinase inhibitors: Emphasis on ponatinib", INTERNATIONAL JOURNAL OF CARDIOLOGY, vol. 316, October 2020 (2020-10-01), AMSTERDAM, NL, pages 214 - 221, XP093018702, ISSN: 0167-5273, Retrieved from the Internet <URL:https://www.sciencedirect.com/science/article/pii/S0167527320307233/pdfft?md5=600d311345c89e743e601e4fa0da0912&pid=1-s2.0-S0167527320307233-main.pdf> DOI: 10.1016/j.ijcard.2020.05.077

## Description

The invention relates to a mouse model demonstrating a pathological state of diastolic heart failure with preserved ejection fraction and method of obtaining it.

Heart failure is a chronic cardiovascular disease that occurs when the heart muscle is unable to pump enough blood to meet the body's oxygen needs. Heart failure is one of the leading causes of death worldwide. The current classification of heart failure is based on the measurement of the percentage of total blood that is pumped out of the left ventricle during a single cardiac cycle and. Correspondingly, a distinction is made between heart failure with reduced ejection fraction (HFrEF) and heart failure with preserved ejection fraction (HFpEF). Although there are several treatment regimens for HFrEF that increase survival and reduce hospitalisation rates, there is no effective targeted therapy for HFpEF. Patients with HFpEF symptoms do not benefit from the classic treatment regimens used to treat HFrEF, and a search for new therapeutic targets in HFpEF is needed.

Heart failure, both HFrEF and HFpEF, is a systemic disease and therefore the pathological mechanisms and therapeutic efficacy of new compounds cannot be studied in isolated organs or cell cultures, as such, they should be investigated in animal models of HFrEF and HFpEF. Indeed, research into the pathogenesis, new ways of diagnosing and therapy towards heart failure has been successfully conducted in animal models, in particular, in murine models. Several mouse models of heart failure have been developed that can resemble the key clinical manifestations of heart failure, but these are mainly limited to animal models of HFrEF. Currently, the animal models of heart failure are predominantly based on surgical interventions, the administration of pharmacological agents, or the use of an appropriate feeding regimen, which in turn lead to the development of heart failure, as well as combinations of the above methods.

Unlike HFrEF models, HFpEF models are difficult to obtain because they require the application of multiple deleterious factors simultaneously, such as advanced animal age, high-fat diet, pharmacological agents, among others. Therefore, current murine models of HFpEF are almost exclusively based on multiple pathological factors, and when a single deleterious factor is applied, it has to be applied over an extended period of time reaching tens of weeks. Such models, although they exist, have many limitations to their use in testing the efficacy of new therapies.

International patent application WO21263127 A1 discloses a method of inducing heart failure with preserved ejection fraction (HFpEF) in a laboratory animal. The disclosed method comprises administering a composition as defined in this application to a laboratory animal. The document also discloses a method of inducing heart failure by additionally feeding the animals a diet containing about 50% - 50% (wt/wt) of a standard diet and a non-standard diet, the standard diet containing about 33% carbohydrate, about 3.9% fat, about 16.1% protein, about 0.4% salt and no cholesterol; the non-standard diet containing about 39.8% carbohydrate, about 40-44.1% fat, about 16.2% protein, about 2% cholesterol, about 0.5-0.7% sodium chloride. In the execution example, the administration of the diet lasts from 30 days to 90 days to induce and maintain high blood pressure. In the application example, elevated exercise intolerance and/or diastolic dysfunction (elevated E/A ratio in echocardiographic measurements) were observed in addition to elevated blood pressure.

International patent application WO2017218418 A1 discloses a method of inducing heart failure with preserved ejection fraction (HFpEF) in a laboratory animal. The disclosed method comprises administering a composition as defined in this application to a laboratory animal. The document also discloses a method of inducing heart failure by additionally administering to the animals a diet containing 60% kcal derived from fat compared to a standard diet, and a combination of administration of the diet and 0.05-0.1% methyl-Nw-nitro-L-arginine ester chloride. In the application example, the administration of the diet lasts from 35 days to 105 days to induce and maintain reduced contractility and left ventricular hypertrophy. In the application example, left ventricular hypertrophy, increased exercise intolerance and/or diastolic dysfunction (increased E/A ratio in echocardiographic measurements, decreased left ventricular global maximum longitudinal strain as determined by echocardiography) were observed.

Analogously to the above method of inducing heart failure is revealed in an article by Withaar et al. (Cardiovascular Research 2021, 117(9):2108-2124), which describes the use of a combination of dietary HFD or administration of angiotensin II (instead of L-NAME) in elderly mice, i.e. aged 18-22 months. In contrast, International Application No. WO21263127 A1 presents the administration of Angiotensin II as a means of inducing diastolic heart failure in rats in alternative to the rat model with sodium chloride administration (deoxycorticosterone acetate-salt rats, DOCA, rats with increased susceptibility to hypertension induced by chronic sodium chloride administration in drinking water).

On the other hand, Chinese patent application CN110038016 A discloses a method of inducing heart failure with preserved ejection fraction (HFpEF) in mice aged 2-4 months administered a high-fat diet. The disclosed method comprises the administration of a high-fat diet and deoxycorticosterone pivalate to the mice. The diet disclosed in the application contains 50-70% fatty acids, 15-25% carbohydrates, 15-25% proteins. Deoxycorticosterone pivalate at a dose of 60-90 mg/kg is administered 10-12 months after the high-fat diet started.

In addition, epidemiological data suggest that there is an increasing number of patients who develop heart failure as a result of prior cancer treatment. In these patients, heart failure may develop independently of other classical risk factors used in stratifying cardiovascular risk in the general population.

Tyrosine kinase inhibitors (TKIs) are a class of therapeutic agents that inhibit tyrosine kinases. A number of TKIs targeting various tyrosine kinases have been shown to be effective anti-cancer and anti-leukemic agents. TKIs carries a significant risk of adverse cardiovascular effects, including, but not limited to, the development of heart failure.

Risto Kerkelä et al. in the article *Cardiotoxicity of the cancer therapeutic agent imatinib mesylate* (Kerkelä R et al. Nat Med 2006, 12, 908-916) discloses a mouse that is administered with the tyrosine kinase BCR-ABL inhibitor, imatinib. The mice receive between 50 and 200 mg/kg/day for 3 or 6 weeks and have cardiac dysfunction. This document discloses murine model of Heart Failure with Reduced Ejection Fraction (HFrEF) as demonstrated in Tabel 1 in D1 however it lacks the information on the age of mice.

The aim of the invention was to develop a method of obtaining a model demonstrating a pathological state of diastolic heart failure with preserved ejection fraction, which was different from known state-of-the-art methods and would produce reproducible results with a reduced intervention time from tens to weeks and eliminate the need for multiple hits to induce HFpEF pathology simultaneously, thereby increasing the controllability and reproducibility of the mouse model of HFpEF. An additional goal was to eliminate the use of animals of advanced age that spontaneously develop HFpEF themselves, reducing the costs associated with housing the animals, thereby reducing the cost of the experiments as well as the time to complete them.

When conducting experiments on laboratory mice administered TKIs, a pathological state of diastolic heart failure with preserved ejection fraction was unexpectedly observed.

Thus, the object of the invention is a method of obtaining a mouse model exhibiting a pathological state of diastolic heart failure with preserved ejection fraction (HFpEF), the method comprising administering to mice a tyrosine kinase inhibitor compound at a dose of at least 1 mg/kg for a period for 2 to 4 weeks, preferably for 4 weeks, wherein said mice are no more than 24 weeks old at the start of the administration of TKI compound, preferably from 8 to 12 weeks old, wherein tyrosine kinase inhibitor is a tyrosine kinase inhibitor having the oncological therapeutic target of BCR-ABL tyrosine kinase, and belonging to the 2nd or 3rd generation of tyrosine kinase inhibitors for the treatment of Chronic Myeloid Leukemia (CML), when in the method of the invention the administered compound is a tyrosine kinase inhibitor selected from the following group:
(a) 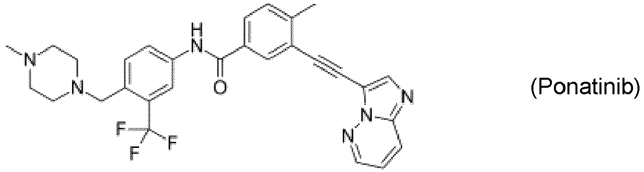 wherein Ponatinib is administered at a dose of 1 to 15 mg/kg, more preferably at a dose of 1 to 10 mg/kg, most preferably at a dose of 1 mg/kg,
(b) 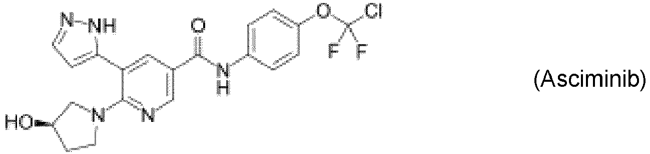 wherein Asciminib is administered at a dose of 1 to 15 mg/kg, more preferably at a dose of 1 to 10 mg/kg, most preferably at a dose of 3 mg/kg,
(c) 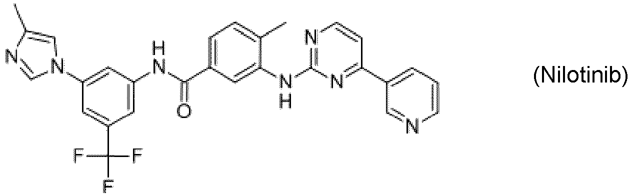 wherein Nilotinib is administered at a dose of 20 to 40 mg/kg, more preferably at a dose of 30 mg/kg.

The invention also includes a mouse model of diastolic heart failure with preserved ejection fraction obtained by the above method.

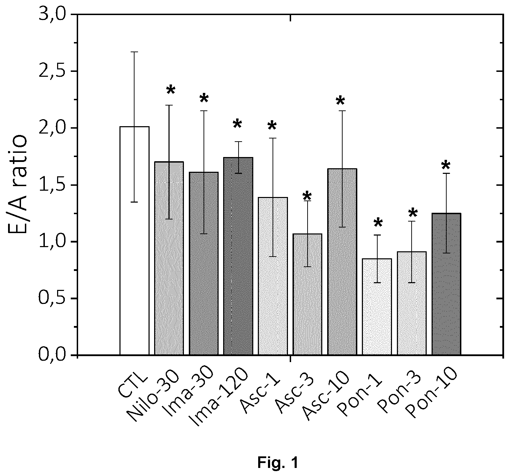

The object of the invention is illustrated in embodiments not limiting the scope of the invention, that is defined by the appended claims, and in a drawing, where the fig. 1 compares the values of the ratio of the left ventricular filling rate in the *early* (E) phase of diastole to the left ventricular filling rate in the *atrial* (A) phase of diastole as a measure of impairment of cardiac diastolic function, for the used TKIs inhibitors.

In the embodiment, a series of experiments were performed in which the following tyrosine kinase inhibitor compounds were administered to mice (male wild-type strain C57/BL6j): Ponatinib at 1, 3 10 mg/kg, Asciminib at 1, 3 and 10 mg/kg, Nilotinib at 30 mg/kg and Imatinib at 30, 120 and 360 mg/kg. These tyrosine kinase inhibitors were administered to mice aged 8-12 weeks for a period of four weeks. This was followed by a cardiac function study using magnetic resonance tomography imaging. During the study, the animals were kept under inhalation anaesthesia using isoflurane at a dose of 1.5-1.75% in a 1:2 mixture of oxygen and air. During the measurement, the animals' condition was monitored using a respiratory sensor, temperature sensor, and ECG system. To assess left ventricular function, a retrospectively gated gradient echo sequence (FLASH) was used. A series of 6-8 imaging slices oriented along the short axis of the heart was obtained to cover the entire left ventricular volume. Based on the obtained imaging data, the cardiac cycle curve, i.e. the change in left ventricular area over time, was calculated according to the protocol described by Tyrankiewicz et al. (NMR in Biomedicine 2016, 29(6):833-840). The following parameters determining left ventricular function were determined from the left ventricular area over time curve:
- *ESV*: *End Systolic Volume,*
- *EDV*: *End Diastolic Volume,*
- *EF*: *Ejection* fraction,
- *SV*: *Stroke Volume (SV=EDV-ESV),*
- *CO*: *Cardiac Output (CO=SV*HR*/*1000,* where *HR is the* heart rate),
- *CI*: *Cardiac* index (*CI*=*SV*/*{9.822* × *[body weight (g)]2*/*3}*)

Systolic kinetics was assessed as the slope in the initial phase of systole (as the slope of the curve tangent to successive points marking the change in the left ventricular area during the first approximately 30% of the cycle), and filling kinetics was assessed as the slope in the initial filling phase of the left ventricle (in the first approximately 30% of the ventricular inflow phase) referred to as ER (Ejection Rate) and FR (Filling Rate), respectively. The corresponding left ventricular kinetic times: ejection time (ET), filling time (FT), isovolumic_relaxation time (IVRT), and isovolumic contraction time (IVCT) were obtained from the curve by manually marking consecutive parts of the cardiac cycle. The early filling rate (E) and atrial phase (A) were calculated from the derivative of the cardiac cycle curve. Deformability (strain) analysis was performed using MR tagging imaging, peak radial strain and peak circumferential strain were calculated using the SPAMM algorithm.

Parameters describing cardiac systolic function are ejection fraction (EF), stroke volume (SV), cardiac output (CO), end-systolic volume (ESV), ejection rate (ER), and ejection time (ET). On the other hand, parameters describing the diastolic function of the heart are end-diastolic volume (EDV), filling rate (FR), filling time (FT), early phase filling index (E), atrial phase filling index (A) and E/A ratio.

Systolic failure was defined as a significantly reduced ejection fraction (EF) and impairment in at least one of the following additional parameters: stroke volume, cardiac output, end-systolic volume, and ejection rate. Diastolic failure, on the other hand, was defined as a preserved normal ejection fraction (EF) and impaired in at least one of the following parameters: filling rate, early phase filling rate (E), atrial phase filling rate (A), E/A ratio.

The results obtained for the different doses are shown in Table 1. The mean heart rate increased significantly in the Nilotinib 30 mg/kg group of mice and decreased in the Imatinib 30 and 20 mg/kg, Asciminib 1, 3 and 10 mg/kg, Ponatinib 1 and 3 mg/kg groups. Total left ventricular mass increased significantly only in the Ponatinib 3 mg/kg group. End-systolic volume was significantly increased in the Asciminib 3 mg/kg and Ponatinib 1 mg/kg groups, in the other groups it did not differ from the mean as compared to the control group. Importantly, a change in end-systolic volume alone is not sufficient to conclude systolic heart failure (HFrEF). End-diastolic volume was significantly increased in the Asciminib 3 mg/kg group and did not differ in other groups as compared to the control group. The ejection fraction was not significantly altered in any group. In classic systolic failure with reduced ejection fraction, ejection fraction falls to 40-50% compared to controls. Cardiac output was significantly reduced only in the Imatinib 120 mg/kg group, and in the other groups it did not change as compared to the control group. The cardiac index remained unchanged in all experimental groups. The ejection rate remained unchanged in all experimental groups. The filling rate was significantly reduced in the Nilotinib 30 mg/kg, Imatinib 30 and 120 mg/kg, Ponatinib 1, 3 and 10 mg/kg groups. Ejection time and filling time remained unchanged in all experimental groups. The isovolumic relaxation time was significantly reduced in the Asciminib 3 mg/kg group. The isovolumetric contraction time was significantly reduced in the Ponatinib 1, 3 and 10 mg/kg groups. The early phase filling index (*E*) was significantly reduced in the Nilotinib 30 mg/kg, Imatinib 120 mg/kg, Asciminib 10 mg/kg, Ponatinib 1, 3 and 10 mg/kg groups. The atrial phase filling index (A) was significantly increased in the Asciminib 3, 10 mg/kg, Ponatinib 1 and 3 mg/kg groups. The *E*/*A* ratio was significantly reduced in all experimental groups (as shown in fig. 1). The peak circumferential strain was significantly reduced in the Asciminib 3 and 10 mg/kg and Ponatinib 3 mg/kg groups. The peak radial strain was significantly reduced in the Ponatinib 3 mg/kg group.

A particularly significant impairment of diastolic cardiac function (defined by a reduced *E*/*A* parameter with preserved systolic function) was observed in mice in all experimental groups, i.e. when the following compounds constituting TKIs were administered:
a) Nilotinib 30 mg/kg: preserved ejection fraction and impaired filling rate, early filling rate (*E*) and *E*/*A* ratio
b) Imatinib 30 mg/kg: preserved ejection fraction and impaired *E*/*A* ratio
c) Imatinib 120 mg/kg: preserved ejection fraction and impaired filling rate, early phase filling index (*E*) and *E*/*A* ratio
d) Asciminib at 1 mg/kg: preserved ejection fraction and impaired *E*/*A* ratio
e) Asciminib 3 mg/kg: preserved ejection fraction and impaired atrial filling rate (A) and *E*/*A* ratio
f) Asciminib 10 mg/kg: preserved ejection fraction and impaired early filling rate (*E*) and *E*/*A* ratio
g) Ponatinib at 1 mg/kg: preserved ejection fraction and impaired filling rate, early phase filling rate (*E*), atrial filling rate (*A*) and *E*/*A* ratio
h) Ponatinib at 3 mg/kg: preserved ejection fraction and impaired filling rate, early filling rate (*E*), atrial filling rate (*A*) and *E*/*A* ratio
i) Ponatinib at 10 mg/kg: preserved ejection fraction and impaired filling rate, early filling rate (*E*), and *E*/*A* ratio

| **Table 1. Assessment of cardiac function in mice aged 18-20 weeks.** | | | | | | | | | | |
|---|---|---|---|---|---|---|---|---|---|---|
| | | | | | | | | | | |

| | *Control* | *Nilotinib 30* | *Imatinib 30* | *Imatinib 120* | *Asciminib 1* | *Asciminib 3* | *Asciminib 10* | *Ponatinib 1* | *Ponatinib 3* | *Ponatinib 10* |
|---|---|---|---|---|---|---|---|---|---|---|
| Body weight [g] | 31 ± 1.2 | 27.2 ± 1.5* | 28.6 ± 0.9* | 27 ± 1.2* | 28.9 ± 1.9 | 28.4 ± 1.7 | 29.2 ± 1.4 | 28.4 ±1.8 | 28.9 ± 1.9 | 28.8 ± 1 \| |
| Average Heart Rhythm [beats/minute] | 568 ± 14 | 593 ± 18 | 537 ± 37* | 503 ± 26* | 528 ± 30 | 531 ± 25 | 535 ± 22 | 528 ± 24 | 528 ± 30 | 533 ± 25 |
| Left ventricle mass [mg] | 79.3 ± 5.3 | 83.3 ± 8 | 83.1 ± 9.3 | 85.1 ±8.6 | 86.7 ± 6.5 | 87.3 ± 6.7 | 86.9 ± 9.5 | 83.7 ±5.1 | 90.2 ± 1.4* | 88.4 ± 9.6 |
| End-systolic volume[µl] | 18.5 ± 2 | 16.5 ± 2.2 | 19.2 ± 5.3 | 18.1 ±4.2 | 23.2 ± 6 | 27.4 ± 4* | 17.4 ± 3.1 | 26.9 ±4.3* | 27 ± 6.9 | 23.6 ± 8.3 |
| End-diastolic volume[µl] | 59.3 ± 5.2 | 52.4 ± 9.6 | 58.5 ± 7.7 | 58.7 ±8.1 | 68.6 ± 6.8 | 72.1 ± 6.7* | 58.2 ± 2.8 | 71.9 ± 8 | 67.9 ± 8 | 63.1 ± 17.7 |
| Stroke volume [µl] | 40.9 ± 4 | 38.7 ± 11.1 | 39.3 ± 4.5 | 40.5 ± 4.7 | 45.4 ± 5.5 | 44.7 ± 4.5 | 40.9 ± 2.4 | 45 ± 6.3 | 40.9 ± 3.8 | 39.6 ± 10.7 |
| Ejection fraction [%] | 66.8 ± 2.5 | **67.8 ± 6.9** | **67.6 ± 6** | **69.3 ± 3.5** | **66.4 ± 6.7** | **63.1 ± 3.7** | **70.2 ± 4.5** | **62.5 ± 4.7** | **62.9 ± 1.6** | **62.8 ± 6.2** |
| Cardiac output [ml/min] | 23.2 ± 2.4 | 22.9 ± 6.3 | 21 ± 2.1 | 20.3 ± 2.1* | 22.1 ± 1.7 | 23.7 ± 2.5 | 21.8 ± 1 | 21.2 ± 6.3 | 21.8 ± 1 | 21.2 ± 6.3 |
| Cardiac index [µl/min/cm2 ] | 240 ± 24 | 257 ± 68 | 229 ± 23 | 230 ± 27 | 259.2 ± 29 | 259.1 ± 21 | 235.2 116 | 229.6 ± 68 | 235.2 116 | 229.6 ± 68 |
| Ejection rate [LV/R-R] | 333 ± 30 | 370 ± 43 | 348 ± 43 | 341 ± 28 | 320 ± 22 | 288 ± 29 | 354 ± 34 | 307 ± 14 | 317 ± 9 | 298 ± 31 |
| Filling rate [LV/R-R] | 485 ±88 | **347 ± 37*** | **415 ± 53*** | **305 ± 85*** | 381 ± 130 | 436 190 | 367 ± 112 | 340 ± 47* | 355 ± 67* | 353 ± 74* |
| Election time [% R-R] | 30.5 ± 3.4 | **26.8 ± 3*** | 29.2 ± 4.9 | 30.2 ± 4 | 32.8 ± 2.8 | 35 ± 1.8 | 27.7 ± 3.5 | 34.2 ± 1.7 | 33.5 ± 1.6 | 35.3 ± 2.5 |
| Filling time [% R-R] | 33.4 ± 6.2 | 36.9 ± 2.5 | 37.3 ± 7.5 | 35.9 ± 4.2 | 35 ± 5 | 32.2 ± 3.4 | 35.6 ± 2.6 | 34.2 ±4.5 | 35.6 ± 2.8 | 32.8 ± 5.3 |
| Isovolumetric relaxation time [% R-R] | 20.1 ± 2.1 | 20.7 ± 4.4 | 20.3 ± 2.1 | 19 ± 2.8 | 19.8 ± 4.4 | 15.8 ± 1.8* | 20.6 ± 3.1 | 19.2 ± 2.6 | 19.9 ± 2.5 | 19.8 ± 2.3 |
| Isovolumetric contraction time [% R-R] | 16 ±4.2 | 15.6 ± 1.4 | 13.2 ± 4.7 | 13.9 ± 3.3 | 12.4 ± 3.5 | 16.9 ± 2.4 | 16.1 ± 3.2 | 12.4 ± 3.3* | 11 ± 2.2* | 12.1 ± 2.5* |
| Early phase filling rate E [%/R-R] | 7.2 ± 0.5 | **6.1 ± 0.7*** | 6.3 ± 1.9 | 6.1 ± 0.9* | 6.2 ± 1.4 | 6.3 ± 1.1 | **6.4 ± 0.9*** | **4.8 ± 0.4*** | **5.1 ± 0.9*** | **5.6 ± 1.2*** |
| Atrial phase filling rate A [%(R-R] | 3.9 ± 1.1 | 3.8 ± 0.8 | 3.2 ± 1.4 | 3.6 ± 0.6 | 4.8 ± 1.1 | **6.1 ± 0.8*** | 4.1 ± 0.9 | **6 ± 1.3*** | **5.8 ± 0.8*** | 4.6 ± 0.7 |
| E/A ratio | 2.01 ± 0.66 | **1.7 ± 0.5*** | **1.61 ± 0.54*** | **1.74 ± 0.14*** | **1.39 ± 0.52*** | **1.07 ± 0.29*** | **1.64 ± 0.51*** | **0.85 ± 0.21*** | **0.91 ± 0.27*** | **1.25 ± 0.35⁰** |
| Peak the circumferential strain [%] | -16.9 ±1.7 | -15 ± 1.1 | -16.3 ± 4 | -15.6 ± 2 | -15.5 ± 3 | -13.7 ± 2.2* | -13.7 * 2.2' | -16.3 ± 4 | -12.3 ± 3* | -15.5 ± 3 |
| Peak radial strain [%] | 27.3 ± 3.4 | 23.1 ± 4.8 | 24.1 ± 3.4 | 25.9 ± 5.4 | 22.2 ± 5.3 | 22.1 ± 6.2 | 20.1 ± 7.1 | 24.1 ± 3.4 | 17 ± 8.7* | 22.2 ± 5.3 |
| | | | | | | | | | | |
| Control mice and mice treated with a tyrosine kinase inhibitor for 4 weeks. The number of mice in each group was n = 6. | | | | | | | | | | |
| * - statistically significant change at the significance level of p < 0.1 with respect to the control group. | | | | | | | | | | |

In addition, a comparative study was also conducted using older mice, i.e. aged 68-70 weeks. In this experiment, mice (male wild-type strain C57/BL6j) were administered Nilotinib at a dose of 30 mg/kg for 4 weeks. Cardiac function was assessed using magnetic resonance imaging according to the methodology outlined in the application example above.

The results obtained for the different doses are shown in Table 2. A statistically significant reduction in ejection fraction to 54% ± 7 as compared to 62.5% ± 4.8 in the control group was observed, as well as an impairment in ejection volume, cardiac output, and end-systolic volume compared to the control group. Thus, systolic heart failure was observed in older mice. To obtain a mouse model of diastolic heart failure in mice, it is, therefore, necessary to use young mice up to 24 weeks of age, and the best results are obtained for mice aged 8 weeks.

**Table 2 Assessment of cardiac function in mice aged 68-70 weeks.**

| | *Control* | *Nilotinib 30 mg*/*kg* |
|---|---|---|
| Body weight [g] | 36.9 ± 2.8 | 32.9 ± 1.7* |
| Average Heart Rhythm [beats/minute]. | 528 ± 50 | 485 ± 34 |
| Left ventricle mass [mg] | 93.1 ± 10.5 | 94 ± 8.7 |
| End-systolic volume[µl]. | 24.7 ± 4.4 | 31.2 ± 8.4 |
| End-diastolic volume[µl]. | 66.1 ± 10.3 | 67.8 ± 13.3 |
| Stroke volume [µl]. | 41.4 ± 7.7 | 36.6 ± 9 |
| **Ejection fraction [%]** | **62.5 ± 4.8** | **54 ± 7*** |
| Cardiac output [ml/min] | 21.7 ± 4 | 17.9 ± 5.7 |
| Cardiac index [µl/min/cm²] | 200.6 ± 40 | 177.2 ± 52 |
| Ejection rate [LV/R-R]. | 319 ± 21 | 319 ± 23 |
| Filling rate [LV/R-R] | 471 ± 60 | 342 ± 43* |
| Ejection time [% R-R] | 33.5 ± 1.6 | 34.2 ± 2.3 |
| Filling time [% R-R] | 33.5 ± 5.2 | 35.3 ± 4.7 |
| Isovolumetric relaxation time [% R-R]. | 19.1 ± 2.1 | 18.1 ± 4.9 |
| Isovolumetric contraction time [% R-R] | 14 ± 2.5 | 12.4 ± 4.4 |
| Early phase filling rate E [%/R-R]. | 6.9 ± 0.6 | 5.7 ± 0.8* |
| Atrial phase filling rate A [%/R-R]. | 5.1 ± 1 | 4.4 ± 1.3 |
| E/A ratio | 1.42 ± 0.45 | 1.41 ± 0.61 |
| Peak the circumferential strain [%] | -16.6 ± 2.9 | -16.6 ± 2.1 |
| Peak radial strain [%] | 27.5 ± 2.8 | 25.2 ± 6.9 |
| In control mice and in mice treated with a tyrosine kinase inhibitor for 4 weeks. The number of mice in each group was n=6. * - statistically significant change at a significance level of p < 0.05 with respect to the control group. | | |

## Claims

1. A method of obtaining a mouse model of diastolic heart failure with preserved ejection fraction, the method comprising administering to mice a compound which is a tyrosine kinase inhibitor at an effective dose of at least 1 mg/kg for a period for 2 to 4 weeks, preferably for 4 weeks, wherein the age of mice at the start of the administration of compound being not more than 24 weeks, preferably from 8 to 12 weeks, wherein tyrosine kinase inhibitor is a tyrosine kinase inhibitor having the oncological therapeutic target of BCR-ABL tyrosine kinase, and belonging to **the** 2nd or 3rd generation of tyrosine kinase inhibitors for the treatment of chronic myeloid leukaemia,
wherein the administered compound is a tyrosine kinase inhibitor selected from the following group consisting of:
(a) 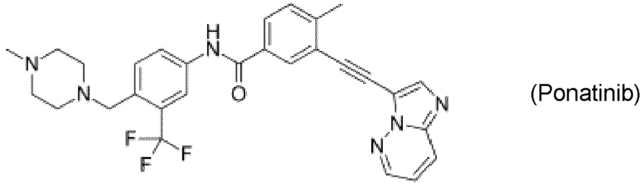 wherein Ponatinib is administered at a dose of 1 to 15 mg/kg, preferably at a dose of 1 to 10 mg/kg, more preferably at a dose of 1 mg/kg,
(b) 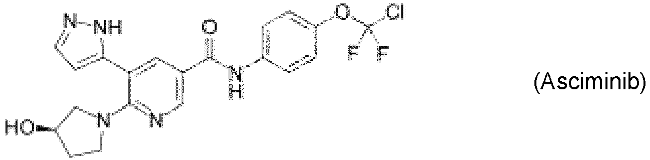 wherein Asciminib is administered at a dose of 1 to 15 mg/kg, preferably at a dose of 1 to 10 mg/kg, more preferably at a dose of 3 mg/kg; and
(c) 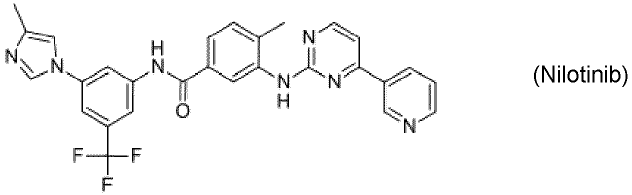 wherein Nilotinib is administered at a dose of 20 to 40 mg/kg, preferably at a dose of 30 mg/kg.

2. A mouse model of diastolic heart failure with preserved ejection fraction obtained by the method according to claim 1.

## Patentansprüche

1. Verfahren zur Herstellung eines Mausmodells der diastolischen Herzinsuffizienz mit erhaltener Auswurffraktion, wobei das Verfahren die Verabreichung einer Verbindung, die ein Tyrosinkinase-Inhibitor ist, an Mäuse in einer wirksamen Dosis von mindestens 1 mg/kg über einen Zeitraum von 2 bis 4 Wochen, vorzugsweise 4 Wochen, umfasst, wobei das Alter der Mäuse zu Beginn der Verabreichung der Verbindung nicht mehr als 24 Wochen beträgt, vorzugsweise 8 bis 12 Wochen, wobei der Tyrosinkinase-Inhibitor ein Tyrosinkinase-Inhibitor ist, der das onkologische therapeutische Ziel der BCR-ABL-Tyrosinkinase hat und zur zweiten oder dritten Generation von Tyrosinkinase-Inhibitoren zur Behandlung der chronischen myeloischen Leukämie gehört,
wobei die verabreichte Verbindung ein Tyrosinkinase-Inhibitor ist, der aus der folgenden Gruppe ausgewählt ist, bestehend aus:
(a) 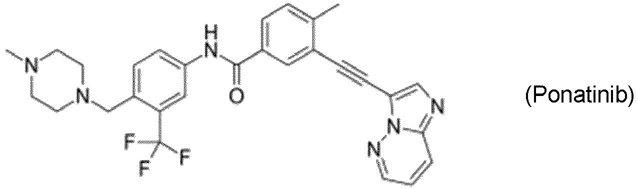 wobei Ponatinib in einer Dosis von 1 bis 15 mg/kg, vorzugsweise in einer Dosis von 1 bis 10 mg/kg, besonders bevorzugt in einer Dosis von 1 mg/kg, verabreicht wird,
(b) 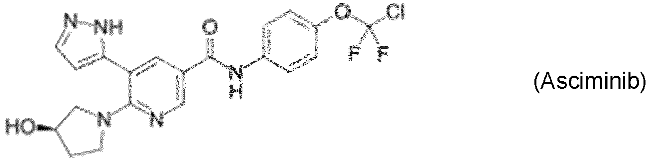 wobei Asciminib in einer Dosis von 1 bis 15 mg/kg, vorzugsweise in einer Dosis von 1 bis 10 mg/kg, besonders bevorzugt in einer Dosis von 3 mg/kg, verabreicht wird, und
(c) 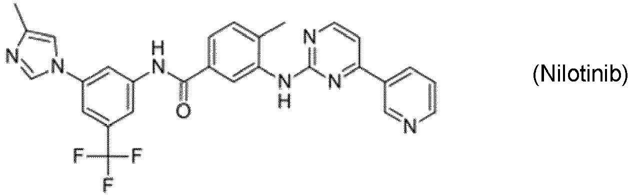 wobei Nilotinib in einer Dosis von 20 bis 40 mg/kg, vorzugsweise in einer Dosis von 30 mg/kg, verabreicht wird.

2. Mausmodell der diastolischen Herzinsuffizienz mit erhaltener Auswurffraktion, erhalten durch das Verfahren nach Anspruch 1.

## Revendications

1. Méthode d'obtention d'un modèle murin d'insuffisance cardiaque diastolique avec fraction d'éjection préservée, la méthode comprenant l'administration à des souris d'un composé qui est un inhibiteur de tyrosine kinase à une dose efficace d'au moins 1 mg/kg pendant une période de 2 à 4 semaines, de préférence pendant 4 semaines, l'âge des souris au début de l'administration du composé n'étant pas supérieur à 24 semaines, de préférence de 8 à 12 semaines, dans lequel l'inhibiteur de tyrosine kinase est un inhibiteur de tyrosine kinase ayant pour cible thérapeutique oncologique la tyrosine kinase BCR-ABL et appartenant à la 2e ou 3e génération d'inhibiteurs de tyrosine kinase pour le traitement de la leucémie myéloïde chronique,
dans lequel le composé administré est un inhibiteur de la tyrosine kinase choisi dans le groupe suivant, composé de
(a) 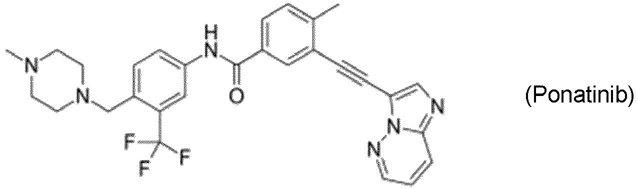 dans lequel le ponatinib est administré à une dose de 1 à 15 mg/kg, de préférence à une dose de 1 à 10 mg/kg, plus préférentiellement à une dose de 1 mg/kg,
(b) 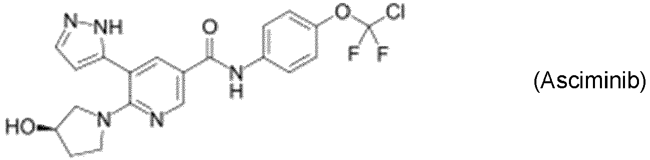 dans lequel l'asciminib est administré à une dose de 1 à 15 mg/kg, de préférence à une dose de 1 à 10 mg/kg, plus préférentiellement à une dose de 3 mg/kg, et
(c) 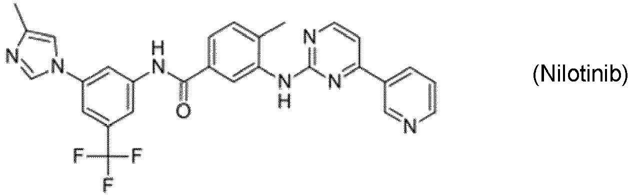 dans lequel le Nilotinib est administré à une dose de 20 à 40 mg/kg, de préférence à une dose de 30 mg/kg.

2. Modèle murin d'insuffisance cardiaque diastolique avec fraction d'éjection préservée obtenu par la méthode selon la revendication 1.
